(19) Europäisches Patentamt
European Patent Office
Office européen des brevets

(11) EP 0 598 770 B1

(12) EUROPÄISCHE PATENTSCHRIFT

(45) Veröffentlichungstag und Bekanntmachung des Hinweises auf die Patenterteilung:
**15.10.1997 Patentblatt 1997/42**

(21) Anmeldenummer: **92916697.3**

(22) Anmeldetag: **31.07.1992**

(51) Int Cl.⁶: **C07D 241/26**, C07D 403/12, C07D 401/12, C07D 405/12, C07D 417/12, A61K 31/495

(86) Internationale Anmeldenummer:
**PCT/EP92/01738**

(87) Internationale Veröffentlichungsnummer:
**WO 93/04048 (04.03.1993 Gazette 1993/06)**

# (54) NEUE PYRAZINDERIVATIVE, IHRE HERSTELLUNG UND VERWENDUNG

NEW PYRAZINE DERIVATIVES, THEIR PREPARATION AND THEIR USE

NOUVEAUX DERIVES DE LA PYRAZINE, LEUR FABRICATION ET LEUR APPLICATION

(84) Benannte Vertragsstaaten:
**AT BE CH DE DK ES FR GB GR IT LI LU NL SE**

(30) Priorität: **16.08.1991 DE 4127026**
**13.09.1991 DE 4130461**

(43) Veröffentlichungstag der Anmeldung:
**01.06.1994 Patentblatt 1994/22**

(73) Patentinhaber:
- **BOEHRINGER INGELHEIM KG**
  **55216 Ingelheim (DE)**
  Benannte Vertragsstaaten:
  **BE CH DE DK ES FR GR IT LI LU NL SE AT**
- **BOEHRINGER INGELHEIM INTERNATIONAL GmbH**
  **55218 Ingelheim am Rhein (DE)**
  Benannte Vertragsstaaten:
  **GB**

(72) Erfinder:
- **KÖPPE, Herbert**
  **D-6507 Ingelheim am Rhein (DE)**
- **SPECK, Georg**
  **D-6507 Ingelheim am Rhein 4 (DE)**
- **STOCKHAUS, Klaus**
  **D-6530 Bingen am Rhein (DE)**

(56) Entgegenhaltungen:
**US-A- 3 313 813**
**US-A- 3 527 758**
**US-A- 3 539 569**

- **CHEMICAL ABSTRACTS, vol. 112, no. 1, 1990, Columbus, Ohio, US; abstract no. 210840f, S. SCHIFFMAN ET AL. 'THE EFFECT OF AMILORIDE ANALOGS ON TASTE RESPONSES IN GERBIL' Seite 47 ; siehe Zusammenfassung & PHISIOL. BEHAVIOUR Bd. 47, Nr. 3, 1990, USA Seiten 435-441**
- **CHEMICAL ABSTRACTS, vol. 111, 1989, Columbus, Ohio, US; abstract no. 208695y, V. PALATY,ED. CRAGOE 'INHIBITION OF MONOAMINE OXIDASE BY ANALOGS OF AMILORIDE.' Seite 13 ; siehe Zusammenfassung & MOL. PHARMACOL. Bd. 36, Nr. 2, 1989, VANCOUVER Seiten 296-301**

Bemerkungen:
Die Akte enthält technische Angaben, die nach dem Eingang der Anmeldung eingereicht wurden und die nicht in dieser Patentschrift enthalten sind.

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 598 770 B1

**Beschreibung**

Die vorliegende Verwendung betrifft neue vierfach substitiuierte Pyrazinderivate, ihre Herstellung nach konventionellen Methoden und ihre Verwendung bei der Herstellung von Arzneistoffen bzw. Arzneimitteln.

Die neuen Verbindungen entsprechen der allgemeinen Formel (I)

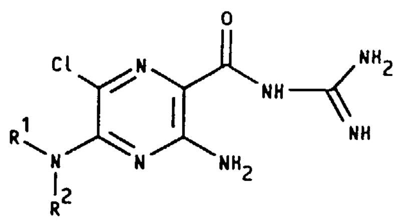

(I)

worin

$R^1$ für H oder einen $C_1$ -$C_8$-Alkylrest steht,

$R^2$ für einen $C_1$-$C_8$-Alkylrest, der substituiert sein kann:

a) durch den Phenylrest, der ein- oder mehrfach durch $C_1$-$C_4$-Alkoxy substituiert sein kann, welches seinerseits durch eine Hydroxylgruppe funktionalisiert sein kann;

b) unabhängig voneinander durch eine oder mehrere $C_1$-$C_4$-Alkoxygruppen, die Hydroxylgruppe oder die Phenoxygruppe, die ihrerseits durch F, Cl, CN, $C_1$-$C_4$-Alkyl, $C_1$-$C_4$-Alkoxy, ($C_1$-$C_4$-Alkoxy)-$C_1$-$C_4$-alkyl ein- oder mehrfach substituiert sein kann;

für eine Amidinogruppe der Formel

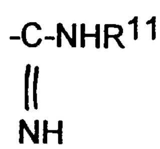

(V),

in der $R^{11}$ einen gegebenenfalls ein- oder mehrfach durch F, Cl oder $C_1$-$C_4$-Alkyl substituierten Phenylrest darstellt; und worin, wenn $R^1$ gleich H ist, $R^2$ außerdem für die Gruppe

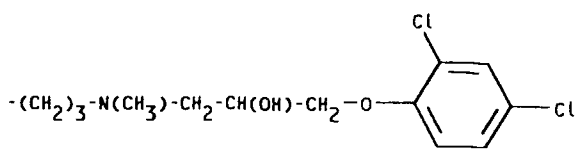

stehen kann;
und
worin die Gruppe

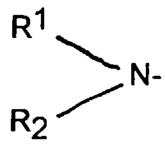

auch einen Piperazinylrest bedeuten kann, der in 4-Stellung substituiert sein kann

a) durch

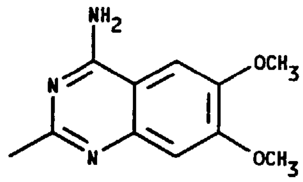

oder

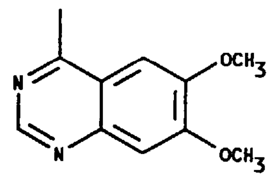

oder

(b) durch einen $C_1$-$C_8$-Alkylrest, der seinerseits unabhängig voneinander substituiert sein kann durch die Hydroxylgruppe oder einen $C_1$-$C_4$-alkoxy- oder ($C_1$-$C_4$-alkoxy)-$C_1$-$C_4$-alkyl-substituierten Phenoxyrest oder einen α-Naphthoxyrest,

jeweils als Basen oder als Salze mit Säuren,
mit der Maßgabe,

(a) daß, wenn $R^1$ Wasserstoff bedeutet, $R^2$ nicht für Alkyl, Benzyl, Methyl-, Fluor- oder Chlorbenzyl, Phenethyl oder Hydroxyethyl oder $HOCH_2$-$(CHOH)_4$-$CH_2$- steht; und
(b) daß, wenn $R^1$ $C_1$-$C_3$-Alkyl ist, $R^2$ nicht für $C_1$-$C_4$-Alkyl steht.

Soweit die Alkylgruppen in den obigen Definitionen bis zu 8 C-Atome enthalten können, sind solche mit bis zu 4 C-Atomen bevorzugt; unter den ($C_1$-$C_4$)-Alkyl- oder -Alkoxygruppen sind diejenigen bevorzugt, die bis zu drei C-Atome enthalten. Als Substituenten in Phenyl-oder Phenoxygruppen sind Alkyl- oder Alkoxyreste mit ein oder auch zwei C-Atomen sowie F und Cl hervorzuheben. Während $R^1$ bevorzugt Wasserstoff oder Methyl ist, steht $R^2$ eher für größere Reste, etwa solche, wie sie in den nachstehenden Tabellen enthalten sind, z.B.

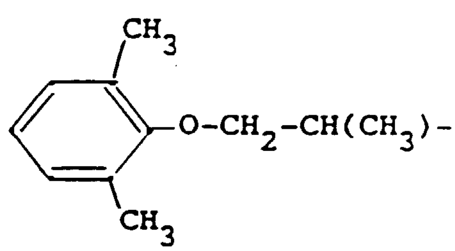

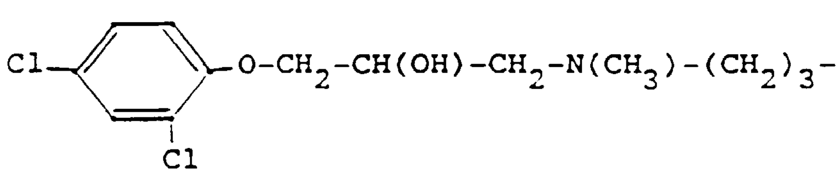

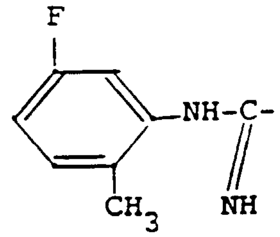

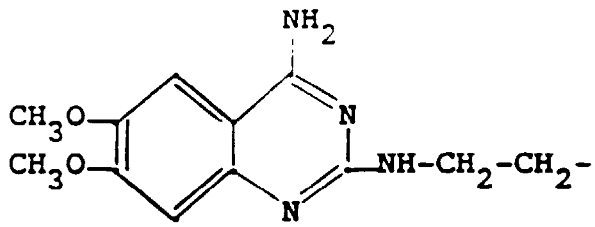

oder $R^1$ und $R^2$ bilden mit dem Stickstoff, an den sie gebunden sind, einen substituierten Heterocyclus, etwa

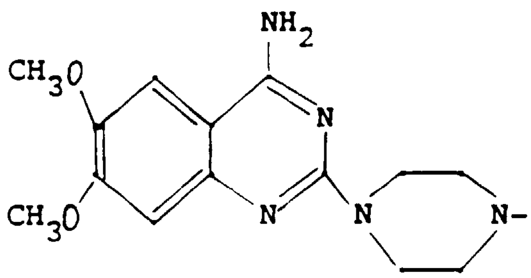

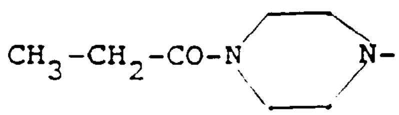

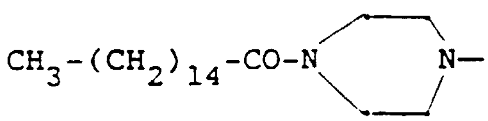

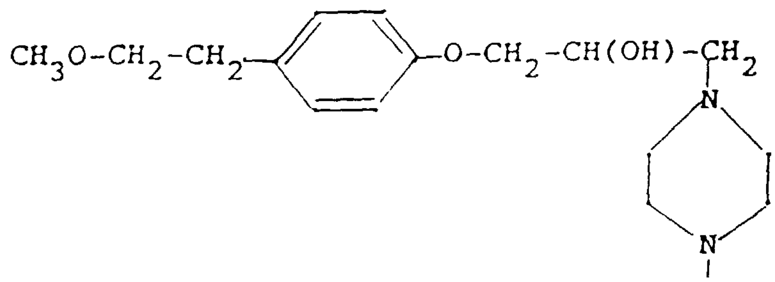

Ähnliche Verbindungen sind aus dem Stand der Technik bekannt, wie z.B. aus den US-Patentschriften 3 313 813, 3 527 758 und 3 539 569 sowie aus Chemical Abstracts Band 111 (1989) Nr. 208 695y und aus Band 112 (1990) Nr. 210 840 f.

Methoden zur Herstellung der neuen Verbindungen sind dem Fachmann bekannt:

1) Man setzt einen Niederalkylester der 3-Amino-5,6-dichlorpyrazin-2-carbonsäure unter wasserfreien Bedingungen mit einem Amin der Formel

$$HNR^1R^2 \qquad (VI)$$

um, in dem $R_1$ und $R_2$ die obige Bedeutung haben,und führt die erhaltene Verbindung der Formel

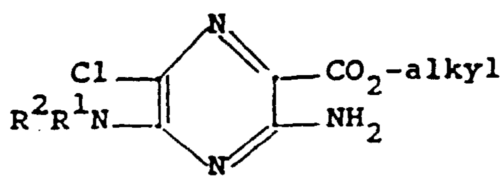

(VII)

(alkyl = $(C_1\text{-}C_4)$-Alkyl)
mit einem Guanidin der Formel

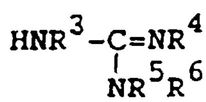

(VIII)

worin $R^3$ bis $R^6$ die obige Bedeutung haben, in das gewünschte Endprodukt über.

Die Umsetzung des 3-Amino-5,6-dichlorpyrazin-2-carbonsäureesters mit der Aminkomponente VI zu den Zwischenverbindungen der allgemeinen Formel VII geschieht nach literaturbekannten Verfahren in einem inerten Lösungsmittel bei erhöhter Temperatur in Gegenwart eines Säurefängers. Die Aminkomponente wird i.a. in äquimolaren Mengen eingesetzt. Es ist aber auch möglich, die Aminkomponente als Säurefänger und Lösungsmittel zu verwenden. Als Lösungsmittel bevorzugt werden jedoch Dimethylformamid und Dimethylsulfoxid oder Gemische davon. Di Reaktionstemperatur ist unkritisch. In der Regel wird die Umsetzung je nach Reaktionsfähigkeit des eingesetzten Amins in einem Temperaturintervall von 80 - 100°C durchgeführt. Unter diesen Bedingungen ist die Reaktion nach ca. 1 - 2 Stunden beendet. Als Säurefänger finden organische und anorganische Basen Verwendung. Bevorzugt wrden tertiäre Amine wie Pyridin, N-Methylpiperidin, Dimethylanilin oder Triethylamin verwendet.

Die auf diese Weise hergestellten 3,5-Diaminopyrazincarbon-säureester der Formel VII können gewünschtenfalls einer Nachbehandlung unterworfen werden, z.B. einer Acylierungs- oder Alkylierungsreaktion.

Die Acylierung erfolgt nach bekannten Verfahren z.B. durch Umsetzung eines Diamins der Formel VII mit einem reaktiven Carbonsäurederivat.

Die Alkylierung findet beispielsweise Anwendung bei der Herstellung entsprechender Phenoxypropanolaminderivate. Dazu wird eine Diaminoverbindung der Substruktur IX der Formel VII:

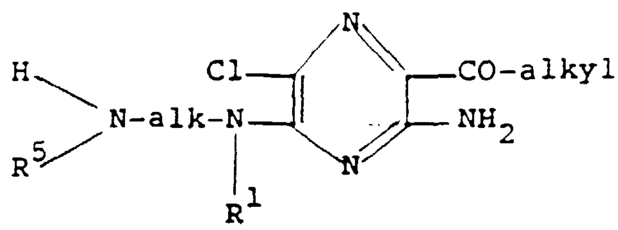

(IX)

(alk = ($C_1$-$C_8$)-Alkylen, alkyl, $R^5$ wie oben) in einem geeigneten Lösungsmittel z.B. mit einem Phenoxypropylenoxid der allgemeinen Formel

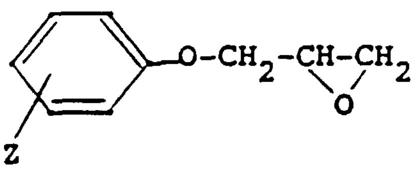

(X)

Z = ($C_1$-$C_4$)-Alkoxy,
($C_1$-$C_4$)-Alkozy-($C_1$-$C_4$)-alkyl
bei Raumtemperatur oder wenig erhöhter Temperatur zur Reaktion gebracht. Im allgemeinen ist die Umsetzung in etwa 0,5 - 1 h beendet.

Bevorzugt wird jedoch der oben beschriebene direkte Syntheseweg.

Zur Herstellung der Endprodukte der Formel I werden die 3,5-Diaminopyrazincarbonsäureester der Formel VII in einem geeigneten Lösungsmittel in der Wärme mit einem Guanidin der Formel VIII umgesetzt. Als Lösungsmittel eignen sich besonders einfache Alkohole. Bevorzugt wird die Umsetzung in Methanol bei Siedehitze durchgeführt. Unter diesen Bedingungen ist die Umsetzung in de Regel nach ca. 30 - 90 Minuten vollständig.

2) Umsetzung eines Pyrazinderivats der Formel

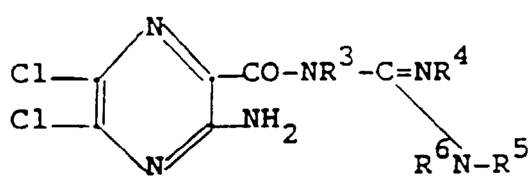

(XI),

in der $R^3$ bis $R^6$ die obige Bedeutung haben, mit einem Amin der Formel

$$HNR^1R^2 \qquad (VI),$$

in der $R^1$ und $R^2$ die obige Bedeutung haben.

Die Umsetzung erfolgt bevorzugt bei erhöhter Temperatur in einem polaren, möglichst wasserfreien Lösungsmittel, z.B. Dimethylformamid, Dimethylsulfoxyd.

Die Ausgangsverbindungen der Formel XI werden nach üblichen Verfahren erhalten. Sie können z.B. entsprechend dem Verfahren 1) erhalten werden, wenn Pyrazincarbonsäureester eingesetzt werden, die analog den Estern der Formel VII gebaut sind, jedoch in 5-Stellung statt $NR^1R^2$ ein Chloratom enthalten.

Die Verbindungen der Formel I sind als Wirkstoffe in Arzneimitteln verwendbar oder können als Zwischenprodukte zur Herstellung solcher Wirkstoffe Verwendung finden. Unter anderem hemmen die neuen Verbindungen den $Na^+/H^+$- und den $Na^+/Li^+$-Austausch. Die erfindungsgemäßen Wirkstoffe können als Antihypertensiva, Mucolytika, Diuretika und Cancerostatika benutzt werden; sie sind ferner anwendbar bei Krankheiten, die im Zusammenhang mit Ischämien stehen (Beispiele: cardiale, cerebrale, gastrointestinale, pulmonale, renale Ischämie, Ischämie der Leber, Ischämie der Skelettmuskulatur). Entsprechende Krankheiten sind beispielsweise coronare Herzkrankheit, Angina pectoris, Embolie im Lungenkreislauf, akutes oder chronisches Nierenversagen, chronische Niereninsuffizienz, Hirninfarkt, chronische Durchblutungsstörungen des Hirns. Bei der Reperfusion des ischämischen Herzens (z.B. nach einem Angina-pectoris-Anfall oder einem Herzinfarkt) können irrevesible Schädigungen an Cardiomyocyten in der betroffenen Region auftreten. Die erfindungsgemäßen Verbindungen können u. a. in einem solchen Fall zur Cardioprotektion benutzt werden.

In das Anwendungsgebiet Ischämie ist auch die Verhinderung von Schäden einzubeziehen, die als Folge von Minderdurchblutung bei Transplantationen auftreten können.

Zur Anwendung der Wirkstoffe eignen sich übliche Formulierungsarten, etwa Tabletten, Dragees, Kapseln, Granulate, Injektionslösungen, ggf. auch nasal applizierbare Zubereitungen, wobei die Wirksubstanz in einer Einzelgabe im allgemeinen in einer Menge von 1 bis 200 mg, vorzugsweise 20 - 100 mg zugegen ist. Die Herstellung dieser Arzneimittelformen erfolgt in an sich bekannter Weise.

## Beispiele

1. Tabletten (Zusammensetzung)

| | |
|---|---|
| Verbindung nach Beispiel | 40,0 mg |
| Maisstärke | 144,0 mg |
| sek. Calciumphosphat | 115,0 mg |
| Magnesiumstearat | 1,0 mg |
| | 300,0 mg |

2. Gelatinekapseln

Der Inhalt einer Kapsel besteht aus 50,0 mg einer Verbindung gemäß der Erfindung und 150,0 mg Maisstärke.

Anmerkung: Unter "Form" ist in den nachstehenden Tabellen angegeben, ob es sich um ein Salz (HCl = Hydrochorid, 2.HCl = Dihydrochlorid usw) oder um die Base (BS) handelt.

## Beispiel 1

a) 3-Amino-6-chlor-5-(2-[1-(2,6-dimethyl-phenoxy)]-propylamino)-pyrazin-2-carbonsäuremethylester

4,44 g (20 mmol) 3-Amino-5,6-dichlorpyrazin-2-carbonsäuremethylester, 3,6 g (20 mmol) 2-Amino-1-(2,6-dimethylphenoxy)propan und 2,2 g (22 mmol) Triethylamin werden in 40 ml wasserfreiem Dimethylformamid 1 1/2 Stunde auf 95 - 100°C erhitzt. Nach dem Abdestillieren des Lösunsmittels im Vakuum wird der Rückstand an Kieselgel (Eluens: Essigester:Isopropanol:$NH_3$ (70:30:1) gereinigt.

Ausbeute 7,3 g

b) N-Amidino-3-amino-6-chlor-5-(2-[1-(2, 6-dimethylphenoxy)]propylamino)pyrazin-2-carboxamid-hydrochlorid

7,3 g (0,02 mmol) Pyrazincarbonsäureester aus Beispiel 1a) in 50 ml Methanol gelöst, werden mit 80 ml einer 1-molaren methanolischen Guanidinlösung 45 Minuten am siedenden Wasserbad erhitzt. Das Lösungsmittel wird im Vakuum abdestilliert, der Rückstand über eine Kieselgelsäule (Eluens:
Essigester:Isopropanol:$NH_3$ (70:30:5) gereinigt, und das Hydrochlorid des Endproduktes hergestellt.

Ausbeute 4,9 g; Fp. 267 - 270°C.

## Beispiel 2

a) 3-Amino-6-chlor-2-[N'-(5-fluor-2-methyl)phenyl]guanidino-pyrazin-2-carbonsäuremethylester

13,3 g (60 mmol) 3-Amino-5,6-dichlorpyrazin-2-carbon- säuremethylester, 15 g (90 mmol) (5-Fluor-2-methyl)-phenylguanidin und 6 g Triethylamin werden in 100 ml DMSO 2 Stunden bei 90°C gerührt. Nach dem Abkühlen wird mit 100 ml $CH_2Cl_2$ versetzt. Unter Kühlen werden 100 ml Wasser zugetropft, die $CH_2Cl_2$-Phase wird abgetrennt, die Wasserphase mit 150 ml $CH_2Cl_2$ extrahiert. Die gesammelten $CH_2Cl_2$-Phasen werden mit wenig Wasser ge-

waschen, über $MgSO_4$ getrocknet und eingeengt. Der Rückstand wird über Kieselgel (Eluens: Essigester: Isopropanol (95 : 5) gereinigt.
Ausbeute: 12,6 g

b) N-Amidino-3-amino-6-chlor-2-[N'-(5-fluor-2-methyl)phenyl]guanidino-pyrazin-2-carboxamid
5,5 g Pyrazincarbonsäure-2-methylester aus Beispiel 2a) werden in 25 ml wasserfreiem Dimethylformamid gelöst, mit 50 ml einer 1,2 molaren methanolischen Guanidinlösung versetzt und 30 Minuten am Rückfluß gekocht. Danach wird das Methanol abdestilliert und der Rückstand an Kieselgel (Eluens: Essigester: Isopropanol:$NH_3$ (70: 30:5)) gereinigt und das Umsetzungsprodukt in das Hydrochlorid überführt.
Ausbeute: 8,7 g; Fp. 242°C.

<u>Beispiel 3</u>

N-Amidino-3-amino-6-chlor-5-(4-[2-(3-methoxyphenoxy)ethyl]-1-piperazinyl)-pyrazin-2-carboxamiddihydrobromid
858 mg (3mmol) N-Amidino-3-amino-5,6-dichlorpyrazin-2-carboxamid, 924 (3 mmol) N-[2-(3-methoxyphenoxy)ethyl] piperazin und 1,2 g (12 mmol) Triethylamin werden 75 Minuten auf 95 - 100°C erhitzt. Das Lösungsmittel wird im Vakuum abdestilliert, der Rückstand an Kieselgel gereinigt (Eluens:
Essiqester:Isopropanol:$NH_3$ (7:3:1)), und das erhaltene Umsetzungsprodukt in das Dihydrobromid überführt.
Ausbeute: 1,13 g; Fp: 159 - 162°C.

<u>Beispiel 4</u>

a) 3-Amino-6-chlor-5-[N-(2-pyrrolcarbonylamino)ethyl-N-methylamino]-pyrazin-2-carbonsäuremethylester
2,73 g (10 mmol) 3-Amino-6-chlor-5-[N-(2-amino)ethyl-N-methyl]amino-pyrazin-2-carbonsäureethylester , 1,35 g (10 mmol) l-Hydroxy-lH-benztriazol-hydrat und 1,11 g (10 mmol) Pyrrol-2-carbonsäure werden, in 50 ml wasserfreiem Tetrahydrofuran gelöst, unter Eiskühlung mit 2,06 g (10 mmol) Dicyclohexylcarbodiimid 15 Stunden bei Raumtemperatur gerührt. Das Umsetzungsprodukt wird auf die übliche Weise isoliert und an Kieselgel Eluens: Essigester:Isopropanol (7:3) gereinigt.
Ausbeute: 3,09 g
b) N-Amidino-3-amino-6-chlor-5-[N-[2-(pyrrolylcarbonylamino)ethyl]-N-methyl]aminopyrazin-2-carboxamidhydrochlorid
Herstellung analog Beispiel 1.
3,09 g (10 mmol) Pyrazin-2-carbonsäuremethylester aus Beispiel 3 liefern 1,65 g der Titelverbindung; Fp. 181 - 184°C.

<u>Beispiel 5</u>

N-Amidino-3-amino-6-chlor-5-[4-[(4-amino-6,7-dimethoxy)-2-chinazolinyl]-1-piperazinyl]-pyrazin-2-carboxamiddihydrochlorid
13,1 g (45,3 mmol). N-[(4-Amino-6,7-dimethoxy)-2-chinazolinyl]-piperazin, 10 g (45,3 mmol) 3-Amino-5,6-dichlorpyrazin-2-carbonsäuremethylester und 7 ml Triethylamin werden in 60 ml Dimethylsulfoxid 2 Stunden bei 80°C umgesetzt. Nach dem Abkühlen wird das Reaktionsprodukt durch Zugabe von 100 ml Wasser ausgefällt, abgesaugt, getrocknet und ohne weitere Reinigung nach der in Beispiel 1a) beschriebenen Methode durch Umsetzung mit Guanidin in das Endprodukt überführt.
Ausbeute: 10,5 g; Fp. > 290°C.

Die in den nachstehenden Tabellen aufgeführten Verbindungen können analog den Beispielen und nach den Ausführungen in der Beschreibung erhalten werden. Liegen die Verbindungen als Base vor, ist unter "Form" BS angegeben, anderenfalls die Säure, mit der Salzbildung erfolgte.

Tabelle 1

Verbindungen der Formel

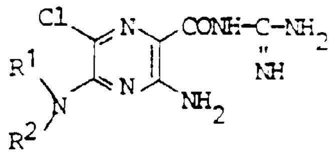

| Nr. | $R^1$ | $R^2$ | Form | Fp [°C] |
|---|---|---|---|---|
| 1 | $CH_3$ | $CH_3$ | HCl | >280 |
| 2 | H | 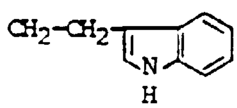 | BS | 188-189 |
| 3 | H | 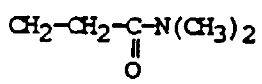 | HCl | 262-264 |
| 4 | H | 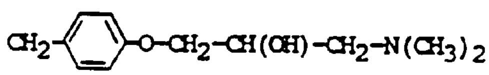 | 2 · HCl | amorph |
| 5 | H | 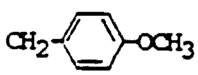 | 2 · HCl | amorph |
| 6 | H | 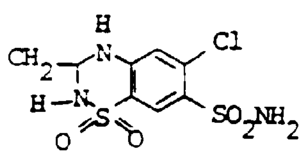 | HCl | 252-253 |
| 7 | H | 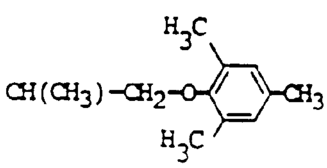 | HCl | 250-253 |
| 8 | H | 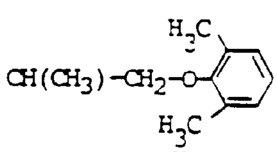 | HCl | 278 |
| 9 | $CH_3$ | 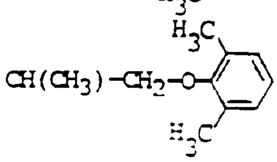 | HCl | 232-235 |

| Nr. | $R^1$ | $R^2$ | Form | Fp. [°C] |
|---|---|---|---|---|
| 10 | H | 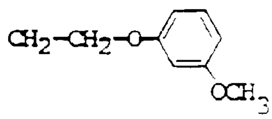 | HCl | 145-148 |
| 11 | H | 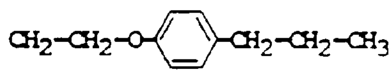 | HCl | 117-119 |
| 12 | H | 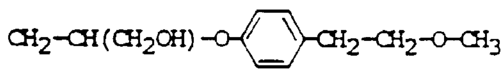 | HCl | 197-200 |
| 13 | H | 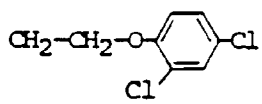 | 2 · HCl | 122-125 |
| 14 | $CH_3$ | 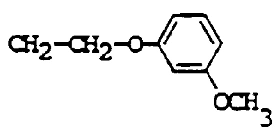 | HCl | 141-143 |
| 15 | H | $CH_2-CH(OCH_3)_2$ | BS | 205-207 |
| 16 | $CH_3$ | 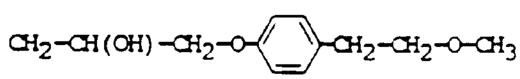 | HCl | amorph |
| 17 | H | 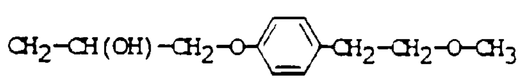 | HCl | 116-119 |
| 18 | $CH_3$ | 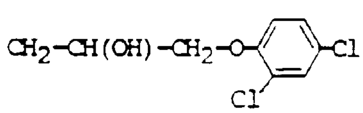 | HCl | 112-115 |
| 19 | $CH_3$ | 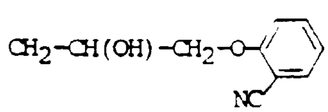 | BS | 207-209 |

| Nr. | $R^1$ | $R^2$ | Form | Fp. [°C] |
|---|---|---|---|---|
| 20 | H | 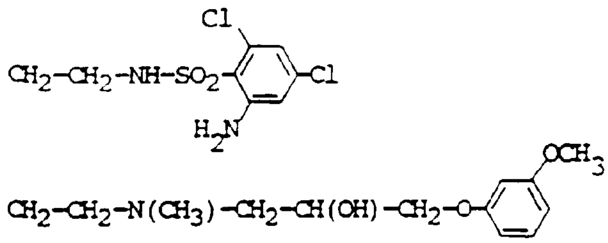 | HCl | 187-191 |
| 21 | H | | 2 · HCl | 118-120 |
| 22 | H | 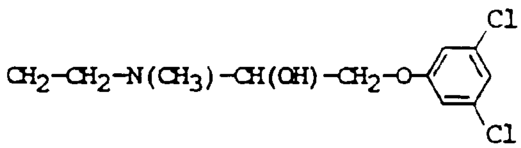 | BS | 83-87 |
| 23 | H | 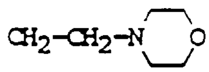 | BS | 200-202 |
| 24 | $CH_3$ | 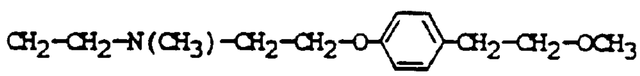 | BS | 95-97 |
| 25 | H | 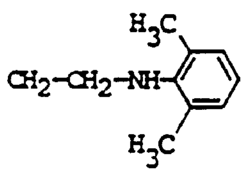 | BS | 210-215 |
| 26 | H | 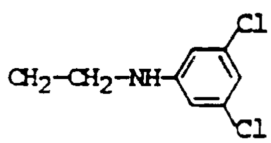 | BS | 225-228 |
| 27 | H | 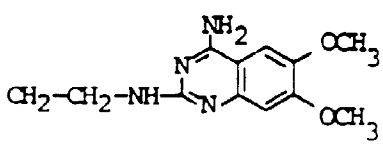 | 2 · HCl | 230-235 |
| 28 | H | 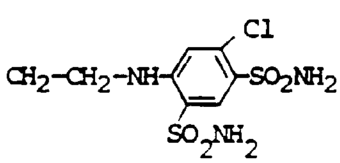 | 2 · HCl | >250 |
| 29 | $CH_3$ | 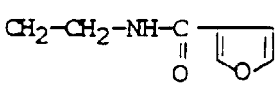 | BS | 140-142 |
| 30 | $CH_3$ | 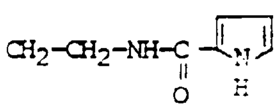 | HCl | 183-186 |

| Nr. | $R^1$ | $R^2$ | Form | Fp (°C) |
|---|---|---|---|---|
| 31 | H | 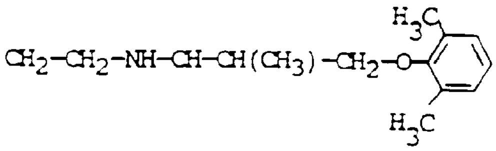 | 2 · HCl | amorph |
| 32 | H | $CH_2-CH_2-NH_2$ | 2 · HCl | >270 |
| 33 | H | 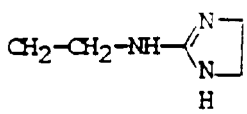 | 3 · HCl | 195-200 |
| 34 | H | 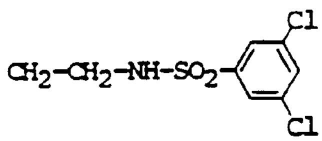 | 2 · HCl | 168-171 |
| 35 | H | 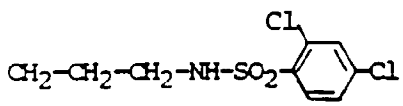 | HCl | 250-252 |
| 36 | H | 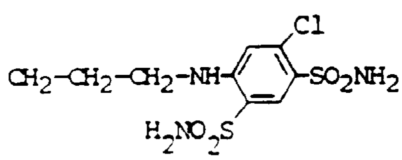 | HCl | 211-215 |
| 37 | H | $CH_2-CH_2-CH_2-N(CH_3)_2$ | 2 · HCl | 268-270 |
| 38 | H | 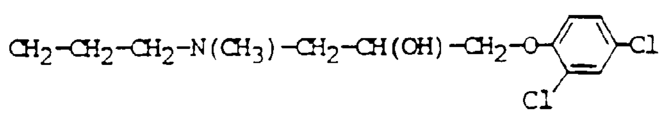 | BS | amorph |
| 39 | H | 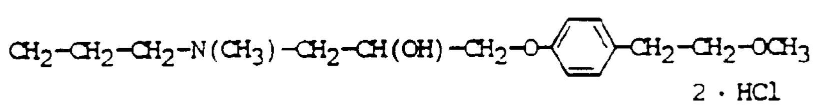 | 2 · HCl | amorph |

Tabelle 2

Verbindungen der Formel

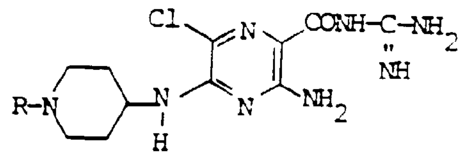

| Nr. | R | Form | Fp.[°C] |
|---|---|---|---|
| 1 | 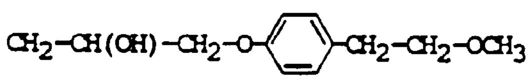 | BS | 184-187 |
| 2 | 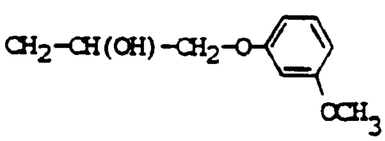 | BS | 163-167 |
| 3 | 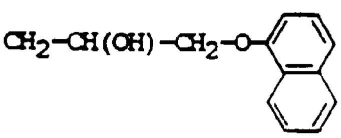 | BS | amorph |
| 4 | 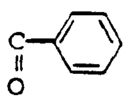 | BS | 187-190 |
| 5 | 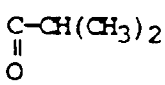 | BS | 218-220 |

Tabelle 3

Verbindungen der Formel

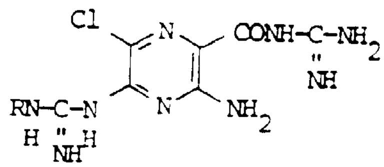

| Nr. | R | Form | Fp. [°C] |
|---|---|---|---|
| 1 | 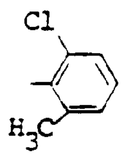 | 2 · HCl | 237-240 |
| 2 | 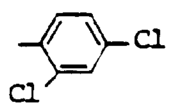 | 2 · HCl | 264-267 |
| 3 | 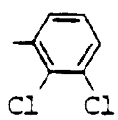 | 2 · HCl | 239-242 |
| 4 | 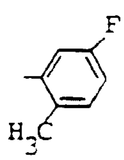 | 2 · HCl | 242 |
| 5 | 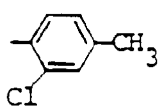 | BS | 177-180 |

Tabelle 4

Verbindungen der Formel

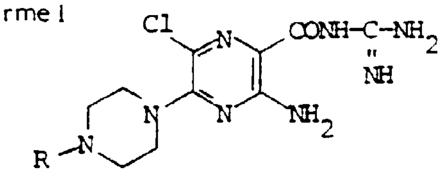

| Nr. | R | Form | Fp. [°C] |
|---|---|---|---|
| 1 | 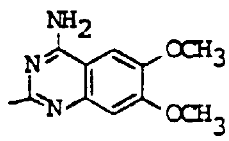 | 2 · HCl | 274 |
| 2 | 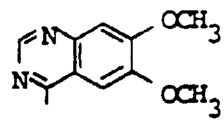 | 2 · HCl | 225-230 |
| 3 | 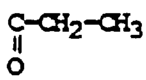 | HCl | 211-213 |
| 4 | 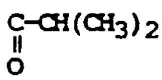 | BS | 143-145 |
| 5 | 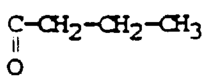 | HCl | 231-233 |
| 6 | 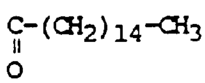 | 2 · HCl | 165-167 |
| 7 | 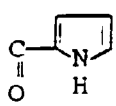 | HCl | 181-184 |
| 8 | 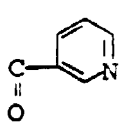 | BS | 210-213 |
| 9 | H | 2 · HCl | >290 |

| Nr. | R | Form | Fp. [°C] |
|---|---|---|---|
| 10 | 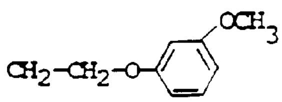 | 2 · HBr | 159-162 |
| 11 | 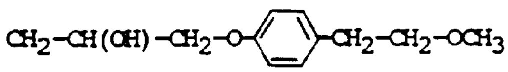 | BS | 180-182 |
| 12 | 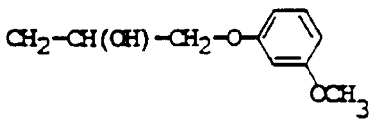 | 2 · HCl | 268-270 |
| 13 | 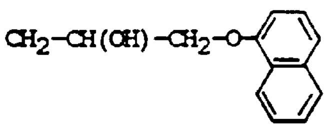 | 2 · HCl | 287-290 |
| 14 | 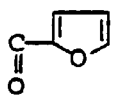 | BS | 196-198 |

## Patentansprüche

1. Verbindungen der allgemeinen Formel

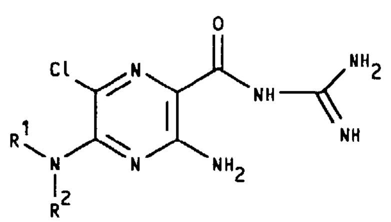

(I)

worin

$R^1$ für H oder einen $C_1$-$C_8$-Alkylrest steht,
$R^2$ für einen $C_1$ -$C_8$-Alkylrest, der substituiert sein kann:

a) durch den Phenylrest, der ein- oder mehrfach durch $C_1$-$C_4$-Alkoxy substituiert sein kann, welches seinerseits durch eine Hydroxylgruppe funktionalisiert sein kann;
b) unabhängig voneinander durch eine oder mehrere $C_1$-$C_4$-Alkoxygruppen, die Hydroxylgruppe oder die Phenoxygruppe, die ihrerseits durch F, Cl, CN, $C_1$-$C_4$-Alkyl, $C_1$-$C_4$-Alkoxy, ($C_1$-$C_4$-Alkoxy)-$C_1$-$C_4$-alkyl ein- oder mehrfach substituiert sein kann;

für eine Amidinogruppe der Formel

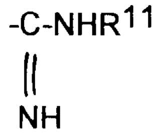

(V),

in der $R^{11}$ einen gegebenenfalls ein- oder mehrfach durch F, Cl oder $C_1$-$C_4$-Alkyl substituierten Phenylrest darstellt; und worin, wenn $R^1$ gleich H ist, $R^2$ außerdem für die Gruppe

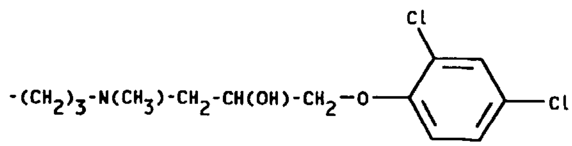

stehen kann;
und
worin die Gruppe

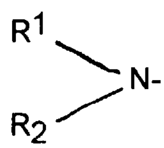

auch einen Piperazinylrest bedeuten kann, der in 4-Stellung substituiert sein kann

a) durch

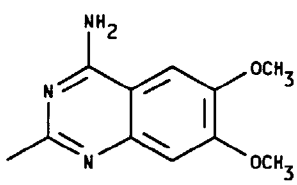

oder

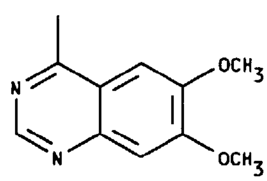

oder

(b) durch einen $C_1$-$C_8$-Alkylrest, der seinerseits unabhängig voneinander substituiert sein kann durch die Hydroxylgruppe oder einen $C_1$-$C_4$-alkoxy- oder ($C_1$-$C_4$-alkoxy)-$C_1$-$C_4$-alkyl-substituierten Phenoxyrest oder einen α-Naphthoxyrest,

jeweils als Basen oder als Salze mit Säuren,
mit der Maßgabe,

(a) daß, wenn $R^1$ Wasserstoff bedeutet, $R^2$ nicht für Alkyl, Benzyl, Methyl-, Fluor- oder Chlorbenzyl, Phenethyl oder Hydroxyethyl oder $HOCH_2$-$(CHOH)_4$-$CH_2$- steht; und
(b) daß, wenn $R^1$ $C_1$-$C_3$-Alkyl ist,
$R^2$ nicht für $C_1$-$C_4$-Alkyl steht.

**2.** N-Amidino-3-amino-6-chlor-5-[4-[(4-amino-6,7-dimethoxy)-2-chinazolinyl] -1-piperazinyl]-pyrazin-2-carboxamid

und seine Säureadditionssalze.

3. N-Amidino-3-amino-6-chlor-5-[N'-(5-fluor-2-methyl)-phenyl]guanidinopyrazin-2-carboxamid und seine Säureadditionssalze.

4. N-Amidino-3-amino-6-chlor-5-[2-[1-(2,6-dimethyl-phenoxy)]propylamino] pyrazin-2-carboxamid und seine Säureadditionssalze.

5. Arzneimittel, gekennzeichnet durch einen Gehalt an einer Verbindung nach einem der Ansprüche 1 bis 4 neben üblichen Hilfs- und/oder Trägerstoffen.

6. Verwendung von Verbindungen nach Anspruch 1, 2, 3 oder 4 bei der Herstellung von Arzneimitteln mit antihypertensiver, mucolytischer, diuretischer, cancerostatischer oder PAF-antagonistischer Wirkung oder Verwendbarkeit bei Krankheiten, die mit Ischämien oder Minderdurchblutung im Zusammenhang stehen.

7. Verfahren zur Herstellung von Verbindungen nach Anspruch 1, 2, 3 oder 4 mittels an sich bekannter Methoden, dadurch gekennzeichnet, daß man

a) einen Pyrazincarbonsäureester der Formel

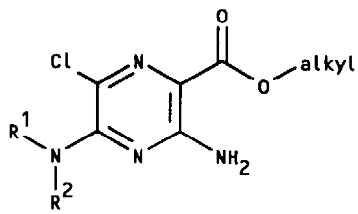

(VII)

(alkyl = ($C_1$-$C_4$)-Alkyl)
mit Guanidin umsetzt
oder daß man

b) ein Pyrazinderivat der Formel

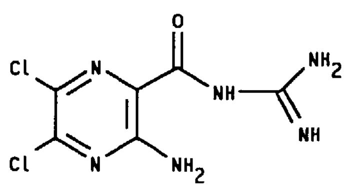

(XI)

mit einem Amin der Formel

$$HNR^1R^2 \qquad (VI)$$

in der $R^1$ und $R^2$ die obige Bedeutung haben, umsetzt
und gewünschtenfalls die erhaltenen Basen der Formel I in Säureadditionssalze überführt.

**Claims**

**1.** Compounds of general formula

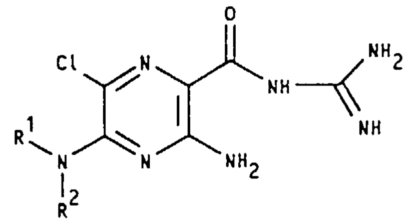

(I)

wherein

$R^1$ represents H or a $(C_{1-8})$alkyl group,
$R^2$ represents a $(C_{1-8})$alkyl group which may be substituted

a) by the phenyl group which is optionally mono-or polysubstituted by a $(C_{1-4})$alkoxy group, which may in turn be functionalised by a hydroxyl group;

b) independently of one another by one or more $(C_{1-4})$alkoxy groups, the hydroxyl or phenoxy group which may in turn be mono- or polysubstituted by F, Cl, CN, $(C_{1-4})$alkyl, $(C_{1-4})$alkoxy or $(C_{1-4})$alkoxy-$(C_{1-4})$alkyl;

and an amidino group of the formula

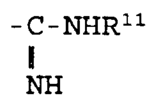

(V) ,

wherein $R^{11}$ represents a phenyl group which is optionally mono- or polysubstituted by F, Cl or a $(C_{1-4})$alkyl group; and wherein, when $R^1$ denotes H, $R^2$ may also represent the group

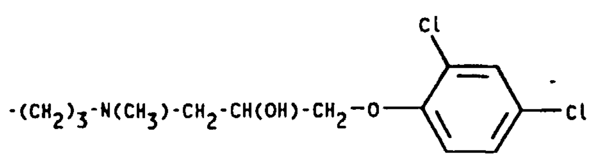

and
wherein the group

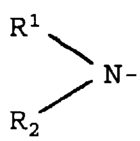

may also denote a piperazinyl group which may be substituted in the 4-position

a) by

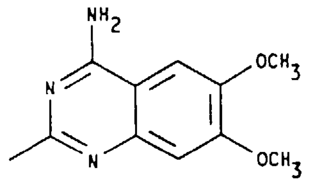 or 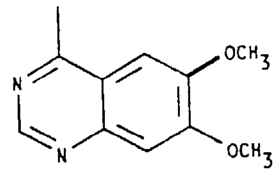

or

(b) by a $C_{1-8}$-alkyl group which in turn may be substituted, independently of each other, by the hydroxy group or by a $C_{1-4}$-alkoxy or $(C_{1-4})$alkoxy-$C_{1-4}$-alkyl-substituted phenoxy group or by an $\alpha$-naphthoxy group,

in the form of bases or salts with acids, with the proviso

(a) that if $R^1$ denotes hydrogen $R^2$ does not denote alkyl, benzyl, methyl-, fluoro- or chlorobenzyl, phenethyl or hydroxyethyl or

$$HOCH_2\text{-}(CHOH)_4\text{-}CH_2\text{-};$$

and

(b) if $R^1$ is $C_{1-3}$-alkyl, $R^2$ does not denote $C_{1-4}$alkyl.

**2.** N-Amidino-3-amino-6-chloro-5-[4-[(4-amino-6,7-dimethoxy)-2-quinazolinyl]-1-piperazinyl]-pyrazine-2-carboxamide and the acid addition salts thereof.

**3.** N-Amidino-3-amino-6-chloro-5-[N'-(5-fluoro-2-methyl)-phenyl]guanidinopyrazine-2-carboxamide and the acid addition salts thereof.

**4.** N-Amidino-3-amino-6-chloro-5-[2-[1-(2,6-dimethylphenoxy)]propylamino]pyrazine-2-carboxamide and the acid addition salts thereof.

**5.** Pharmaceutical compositions, characterised in that they contain a compound according to one of claims 1 to 4 together with conventional excipients and/or carriers.

**6.** Use of compounds according to claim 1, 2, 3 or 4 in the preparation of pharmaceutical compositions having an antihypertensive, mucolytic, diuretic, cancerostatic or PAF-antagonistic activity or those which may be used in diseases connected with ischaemias or reduced blood flow.

**7.** Process for preparing compounds according to claim 1, 2, 3 or 4 by methods known per se, characterised in that

a) a pyrazine carboxylic acid ester of formula

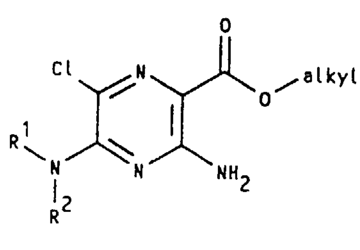

(VII)

(alkyl = ($C_{1-4}$)alkyl)
is reacted with guanidine
or in that

b) a pyrazine derivative of formula

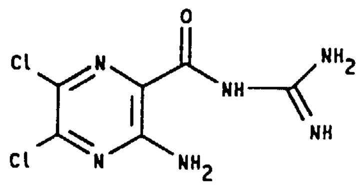

(XI)

is reacted with an amine of formula

$$HNR^1R^2 \qquad (VI)$$

wherein $R^1$ and $R^2$ are as hereinbefore defined

and if desired the bases of formula I obtained are converted into acid addition salts.

**Revendications**

1. Composés de formule générale

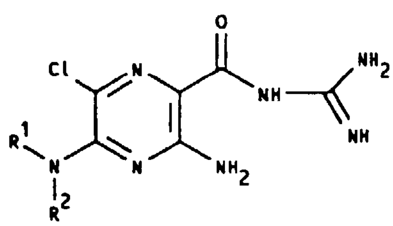

(I)

où

$R^1$ représente H ou un reste alkyle en $C_1$-$C_8$,
$R^2$ représente un reste alkyle en $C_1$-$C_8$ qui peut être substitué :

a) par un reste phényle qui peut être substitué une ou plusieurs fois par alcoxy en C1-C4 qui, pour sa part, peut être fonctionnalisé par un groupe hydroxyle,
b) indépendamment les uns des autres par un ou plusieurs groupes alcoxy en C1-C4, le groupe hydroxyle ou le groupe phénoxy qui, pour sa part, peut être substitué une ou plusieurs fois par F, Cl, CN, alkyle en $C_1$-$C_4$, alcoxy en $C_1$-$C_4$, (alcoxy en $C_1$-$C_4$)-alkyle en $C_1$-$C_4$,
un groupe amidino de formule

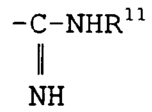 (V)

où $R^{11}$ représente un reste phényle éventuellement substitué une ou plusieurs fois par F, Cl ou alkyle en $C_1$-$C_4$, et où, lorsque $R^1$ est H, $R^2$ peut représenter en outre le groupe

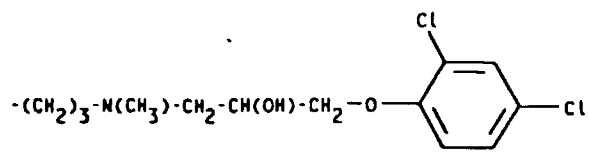

et
où le groupe

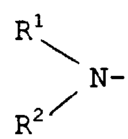

peut aussi représenter un reste pipérazinyle qui peut être substitué en position 4

a) par

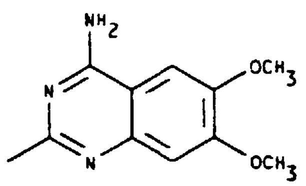

ou

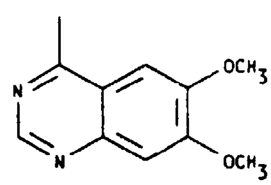

ou
b) par un reste alkyle en $C_1$-$C_8$ qui, pour sa part, peut être substitué indépendamment les des autres par le groupe hydroxyle ou par un reste phénoxy substitué par alcoxy en $C_1$-$C_4$ ou par (alcoxy en $C_1$-$C_4$)-alkyle en $C_1$-$C_4$ ou par un reste $\alpha$-naphtoxy,

dans chaque cas sous forme de bases ou de sels avec des acides,
à condition

(a) que, lorsque $R^1$ représente l'hydrogène, $R^2$ ne représente pas alkyle, benzyle, méthyl-, fluoro- ou chlorobenzyle, phénétyle ou hydroxyéthyle ou $HOCH_2$-$(CHOH)_4$-$CH_2$-, et
(b) que, lorsque $R^1$ représente alkyle en $C_1$-$C_3$, $R^2$ ne représente pas alkyle en $C_1$-$C_4$.

**2.** N-amidino-3-amino-6-chloro-5-[4-[(4-amino-6,7-diméthoxy)-2-quinazolinyl]-1'-pipérazinyl]-pyrazine-2-carboxamide et ses sels d'addition d'acide.

**3.** N-amidino-3-amino-6-chloro-5-[N'-[(5-fluoro-2-méthyl) -phényl] guanidinopyrazine-2-carboxamide et ses sels d'addition d'acide.

**4.** N-amidino-3-amino-6-chloro-5-[2-[1-(2,6-diméthylphénoxy)propylamino]pyrazine-2-carboxamide et ses sels d'addition d'acide.

**5.** Médicament caractérisé par une teneur en un composé selon l'une des revendications 1 à 4 outre des adjuvants et/ou supports habituels.

**6.** Utilisation de composés selon la revendication 1, 2, 3 ou 4 lors de la préparation de médicaments à effet, ou possibilité d'emploi, antihypertenseur, mucolytique, diurétique, cancérostatique ou antagoniste du PAF dans le cas de maladies qui sont associées à des ischémies ou une irrigation sanguine diminuée.

**7.** Procédé de préparation de composés selon la revendication 1, 2, 3 ou 4 au moyen de méthodes connues en soi, caractérisé en ce que

a) on fait réagir un ester d'acide pyrazinecarboxylique de formule

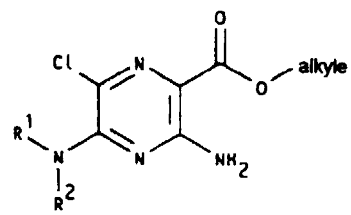

(VII)

(alkyle = alkyle en $C_1$-$C_4$)
avec la guanidine
ou en ce que
b) on fait réagir un dérivé de pyrazine de formule

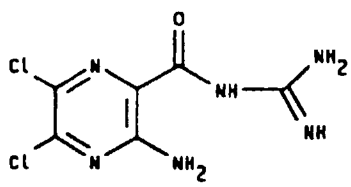

(XI)

avec une amine de formule

$$HNR^1R^2 \qquad (VI)$$

dans laquelle $R^1$ et $R^2$ ont la signification ci-dessus
et, si on le souhaite, on convertit les bases de formule I obtenues en sels d'addition d'acide.